# EUROPEAN PATENT APPLICATION

(11) **EP 4 420 686 A1**
(43) Date of publication of application: **28.08.2024**
(21) Application number: 22883350.5
(22) Date of filing: 05.10.2022
(51) Int. Cl.: A61L 2/10, A23L 3/28, A23L 5/30, C02F 1/32

(54) **FLUID STERILIZATION DEVICE**

(30) Priority: 19.10.2021 JP 2021170841
(71) Applicant: Stanley Electric Co. Ltd., Tokyo 153-8636 (JP)
(72) Inventor: TANAKA Hideaki, Tokyo 153-8636 (JP); KATO Hiroyuki, Tokyo 153-8636 (JP); SHINNO Kazuhisa, Tokyo 153-8636 (JP)
(74) Representative: Klang, Alexander H.
(86) International application number: PCT/JP2022/037247
(87) International publication number: WO 2023/068045

(57) **Abstract**

The present invention has an object of providing a fluid sterilization device that can effectively use wide-angle light of ultraviolet light emitted from a light source unit and allows a fluid to stay in an irradiation range of the ultraviolet light even with a quite simple structure. The fluid sterilization device according to the present invention includes a first flow path portion that extends in an axial direction and allows a fluid to pass from a first end to a second end, a light source unit that is connected to the second end of the first flow path portion and irradiates the fluid with ultraviolet light, a second flow path portion that is opposed to the first flow path portion with the light source unit interposed between the first flow path portion and the second flow path portion, and a third flow path portion that is arranged outside of the light source unit in a radial direction and is configured to allow the fluid flowing through the first flow path portion to flow into the second flow path portion. In the fluid sterilization device, the first flow path portion or the light source unit includes a first communication portion that communicates with the third flow path portion, the second flow path portion or the light source unit includes a second communication portion that communicates with the third flow path portion, and the third flow path portion is any of polymers including polytetrafluoroethylene (PTFE), a perfluoroethylene propene copolymer (FEP), perfluoroalkoxyalkane (PFA), and polypropylene (PP). The first communication portion includes a cut-out communication region that is formed by cutting out a part of a connection portion between the first flow path portion and the light source unit.

## Description

### Technical Field

The present invention relates to a fluid sterilization device.

### Background Art

There are provided various products that irradiate a fluid (for example, drinking liquid, water for food production, or various kinds of cooling water and cleaning water for plants) with ultraviolet light to sterilize it. As inventions related to this, fluid sterilization devices provided at a flow path of a fluid are disclosed, for example, in Patent Literature 1 and 2 described below.

Here, the fluid sterilization device disclosed in Patent Literature 1 includes a straight tubular processing chamber extending from a first end surface to a second end surface, inflow ports and an outflow port that are connected to a sidewall of the processing chamber, and light sources that are arranged near (outside of) the first end surface and the second end surface. Ultraviolet light emitted from the light sources passes through ultraviolet light transmission windows fitted in the first end surface and the second end surface. This configuration allows a fluid flowing near the inflow ports to be irradiated with ultraviolet light of higher intensity than a fluid flowing near the outflow port.

Also, the fluid sterilization device disclosed in Patent Literature 2 includes a first flow path extending in a longitudinal direction, a light source unit that, essentially, is directly in front of the first flow path and irradiates a fluid flowing through the first flow path with ultraviolet light, and a second flow path connected to the first flow path and formed around the light source unit. The fluid flowing through the second flow path flows from a front surface (light emitting surface) side to a rear surface (facing surface to the light emitting surface) of the light source unit.

Furthermore, Patent Literature 2 discloses a mode in which the inside diameter of the flow path at the connection portion between the first flow path and the light source unit is smaller than the inside diameter of the first flow path, and in which the region of the light source unit directly in front of the first flow path is provided with an offset portion recessed toward the inside of the light source unit. As a result, the fluid flowing from the first flow path is collected in the offset portion, and a convection flow (eddy flow) is generated in the fluid collected in the offset portion, whereby the fluid can be irradiated with ultraviolet light for a long period of time.

### Citation List

### Patent Literature

PTL 1: Japanese Patent No. 6571460
PTL 2: Japanese Patent Application Laid-Open No. 2018-8213

### Summary of Invention

### Technical Problem

Meanwhile, the irradiation range of ultraviolet light generated from the light source includes not only the region that is directly in front of the light source but also regions that are laterally irradiated by the light source (ultraviolet light that reaches the regions that are laterally irradiated by the light source may hereinbelow be referred to as "wide-angle light" in some cases). Therefore, by irradiating the fluid with the wide-angle light effectively, the fluid can be sterilized more effectively. However, a method of using the wide-angle light of the ultraviolet light to enhance the ability to sterilize the fluid is not disclosed or suggested in Patent Literature 1.

Also, in the fluid sterilization device in Patent Literature 1, to irradiate the fluid with ultraviolet light including the wide-angle light, the diameter of the processing chamber needs to be widened. However, in this case, the size of the processing chamber (fluid sterilization device) is enlarged.

Also, in the fluid sterilization device in Patent Literature 2, to generate the convection flow in the fluid flowing near the light source unit, a complicated structure must be provided in which the inside diameter of the flow path inside the connection portion is smaller than the inside diameter of the first flow path, and in which the offset portion is provided in the light source unit. This complexity may cause an increase in the cost of manufacturing the fluid sterilization device.

In consideration of the above-described problems, an object of the present invention is to provide a fluid sterilization device that can effectively use wide-angle light of ultraviolet light emitted from a light source unit and allows a fluid to stay in an irradiation range of the ultraviolet light even with a quite simple structure. Solution to Problem

To solve the above-described problems, a fluid sterilization device according to the present invention includes
a first flow path portion that extends in an axial direction and allows a fluid to pass from a first end to a second end,
a light source unit that is connected to the second end of the first flow path portion and irradiates the fluid with ultraviolet light,
a second flow path portion that is opposed to the first flow path portion with the light source unit interposed between the first flow path portion and the second flow path portion, and
a third flow path portion that is arranged outside of the light source unit in a radial direction and is configured to allow the fluid flowing through the first flow path portion to flow into the second flow path portion.

In the fluid sterilization device, the first flow path portion or the light source unit includes a first communication portion that communicates with the third flow path portion,
the second flow path portion or the light source unit includes a second communication portion that communicates with the third flow path portion, and
the third flow path portion is any of polymers including polytetrafluoroethylene (PTFE), a perfluoroethylene propene copolymer (FEP), perfluoroalkoxyalkane (PFA), and polypropylene (PP).

According to the aspect of the present invention, the third flow path portion is provided that communicates with both the first flow path portion and the second flow path portion and is arranged outside of the light source unit in the radial direction. Accordingly, after the fluid flowing through the first flow path portion gets near the light source unit, the fluid flows along the ultraviolet light output surface of the light source unit and heads for the third flow path portion. As a result, the fluid flowing from the first flow path portion into the third flow path portion can be irradiated with the wide-angle light of the ultraviolet light emitted from the light source unit. Also, since the third flow path portion is made of PTFE, which has a high reflectivity with respect to ultraviolet light, the wide-angle light is reflected repeatedly in the third flow path portion. Hence, the fluid flowing through the third flow path portion can be irradiated with the wide-angle light for a long period of time. Consequently, the ability to sterilize the fluid can be enhanced.

Also, a fluid sterilization device according to the present invention includes
a first flow path portion that extends in an axial direction and allows a fluid to pass from a first end to a second end,
a light source unit that is connected to the second end of the first flow path portion and irradiates the fluid with ultraviolet light,
a second flow path portion that is opposed to the first flow path portion with the light source unit interposed between the first flow path portion and the second flow path portion, and
a third flow path portion that is arranged outside of the light source unit in a radial direction and is configured to allow the fluid flowing through the first flow path portion to flow into the second flow path portion.

In the fluid sterilization device, the first flow path portion or the light source unit includes a first communication portion that communicates with the third flow path portion,
the second flow path portion or the light source unit includes a second communication portion that communicates with the third flow path portion, and
the first communication portion includes a cut-out communication region that is formed by cutting out a part of a connection portion between the first flow path portion and the light source unit.

In the aspect of the present invention, the third flow path portion is provided so that it communicates with both the first flow path portion and the second flow path portion and is arranged outside of the light source unit in the radial direction. Accordingly, after the fluid flowing through the first flow path portion gets to the light source unit, the fluid flows along the ultraviolet light output surface of the light source unit and heads for the third flow path portion. As a result, the fluid flowing from the first flow path portion into the third flow path portion can be irradiated with the wide-angle light of the ultraviolet light emitted from the light source unit. Also, the fluid flowing from the first flow path portion into the third flow path portion passes through the first communication portion that includes the cut-out communication region. When this passing of the fluid occurs, the fluid flows into the cut-out communication region in a concentrated manner. As a result, a turbulent flow is generated near the cut-out communication region, and allows the fluid to stay there. Accordingly, the fluid flowing from the first flow path portion into the third flow path portion can be irradiated with ultraviolet light for a long period of time. Consequently, the ability to sterilize the fluid can be enhanced.

Furthermore, it is preferable that, in the fluid sterilization device according to the present invention,
the first communication portion includes a plurality of the cut-out communication regions,
the second communication portion includes a plurality of cut-out communication regions that are formed by cutting out parts of a connection portion between the second flow path portion and the light source unit, and
the cut-out communication regions in the first communication portion and the cut-out communication regions in the second communication portion are arranged alternately in a circumferential direction.

According to the aspect of the present invention, the cut-out communication regions in the first communication portion and the cut-out communication regions in the second communication portion are arranged alternately in the circumferential direction. Hence, the short path of the fluid from the first flow path portion to the second flow path portion via the third flow path portion can be prevented effectively. As a result, it is possible to allow the fluid to stay near the cut-out communication regions in the first communication portion, and the fluid can be irradiated with ultraviolet light for a long period of time. Consequently, the ability to sterilize the fluid can further be enhanced.

Furthermore, it is preferable that, in the fluid sterilization device according to the present invention,
the light source unit further includes an ultraviolet light transmission window portion opposed to the second end of the first flow path unit, and
a width of the ultraviolet light transmission window portion is larger than an inside diameter of the first flow path portion.

According to the aspect of the present invention, the width of the ultraviolet light transmission window portion opposed to the second end of the first flow path unit is larger than the inside diameter of the first flow path portion. Hence, the fluid can also receive ultraviolet light emitted from the ultraviolet light transmission window portion when passing through the first communication portion after getting near the ultraviolet light transmission window portion. As a result, the fluid can be irradiated with ultraviolet light for a longer period of time, and the ability to sterilize the fluid can further be enhanced.

### Advantageous Effects of Invention

According to the present invention, it is possible to provide a fluid sterilization device that can effectively use wide-angle light of ultraviolet light emitted from a light source unit and allows a fluid to stay in an irradiation range of the ultraviolet light even with a quite simple structure. As a result, according to the present invention, the efficiency of sterilization of a fluid can be enhanced.

### Brief Description of Drawings

[Fig. 1] Fig. 1 is a vertical cross-sectional view of a fluid sterilization device according to an embodiment.
[Fig. 2] Fig. 2 is a vertical cross-sectional view of the fluid sterilization device according to the present embodiment.
[Fig. 3] Fig. 3(a) is a cross-sectional view taken along line A-A in Fig. 1 (a lateral cross-sectional view of a first flow path portion), Fig. 3(b) is a cross-sectional view taken along line B-B in Fig. 1 (a lateral cross-sectional view of a third flow path portion), Fig. 3(c) is a cross-sectional view taken along line C-C in Fig. 1 (a lateral cross-sectional view of a second flow path portion), and Fig. 3(d) is a combined cross-sectional view in which the cross-sectional view taken along line A-A and the cross-sectional view taken along line C-C are combined.
[Fig. 4] Fig. 4 is a vertical cross-sectional view of a fluid sterilization device according to Modification Example 1.
[Fig. 5] Fig. 5 is a vertical cross-sectional view of a fluid sterilization device according to Modification Example 2.
[Fig. 6] Fig. 6(a) is a cross-sectional view taken along line D-D in Fig. 5 (a lateral cross-sectional view of the first flow path portion), and Fig. 6(b) is a cross-sectional view taken along line E-E in Fig. 5 (a lateral cross-sectional view of the second flow path portion).
[Fig. 7] Fig. 7 is a vertical cross-sectional view of a fluid sterilization device according to Modification Example 3.

### Description of Embodiments

### [Basic Example]

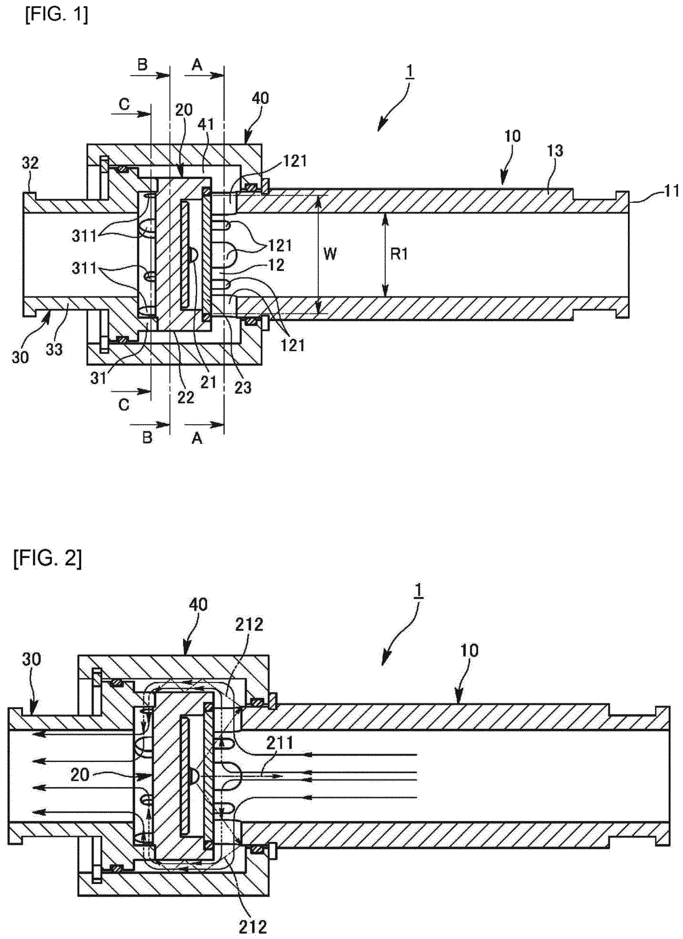

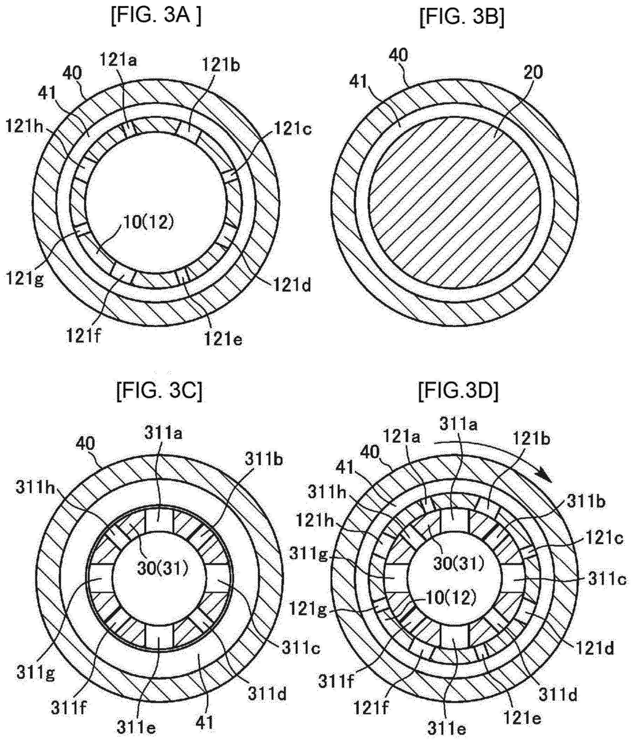

Hereinbelow, a fluid sterilization device according to an embodiment of the present invention will be described in detail with reference to the drawings. First, referring to Figs. 1 to 3, a fluid sterilization device 1 according to the present embodiment will be described. Here, Fig. 1 is a vertical cross-sectional view of the fluid sterilization device 1. Also, Fig. 2 is a vertical cross-sectional view of the fluid sterilization device 1 (a view illustrating a flow of a fluid in the fluid sterilization device 1). Furthermore, Fig. 3(a) is a cross-sectional view taken along line A-A in Fig. 1 (a lateral cross-sectional view of a first flow path portion including a first communication portion (all cut-out communication regions) described below), Fig. 3(b) is a cross-sectional view taken along line B-B in Fig. 1 (a lateral cross-sectional view of a third flow path portion described below), Fig. 3(c) is a cross-sectional view taken along line C-C in Fig. 1 (a lateral cross-sectional view of a second flow path portion including a second communication portion (all cut-out communication regions) described below), and Fig. 3(d) is a combined cross-sectional view in which the cross-sectional view taken along line A-A and the cross-sectional view taken along line C-C are combined.

As illustrated in Fig. 1, the fluid sterilization device 1 according to the present embodiment includes a first flow path portion 10, a light source unit 20 connected to a second end 12 of the first flow path portion 10, a second flow path portion 30 opposed to the first flow path portion 10 with the light source unit 20 interposed between the first flow path portion 10 and the second flow path portion 30, and a third flow path portion 40 arranged outside of the light source unit 20 in the radial direction.

The first flow path portion 10 includes a first end 11, the second end 12, and a tubular sidewall 13 and extends in the axial direction (longitudinal direction). Also, the second end 12 of the first flow path portion 10 is provided with a first communication portion 121 that communicates with the third flow path portion 40. When the fluid flowing through the first flow path portion 10 gets near the second end 12, the fluid passes through the first communication portion 121 and flows through the third flow path portion 40 (flow path 41) (refer to Fig. 2). Here, the first communication portion 121 according to the present embodiment corresponds to a plurality of communication regions (cut-out communication regions) that are formed by cutting out parts of the second end 12 (connection portion to the light source unit 20) in the direction towards the first end 11. The shapes (sizes) of the plurality of communication regions may be equal to, or different from, one another. However, the mode of the first communication portion 121 is not limited to that stated above.

Furthermore, the first flow path portion 10 according to the present embodiment is made of polytetrafluoroethylene (PTFE), but the material is not limited to that stated above. The material for the first flow path portion 10 may be a resin other than PTFE (for example, a perfluoroethylene propene copolymer (FEP) or perfluoroalkoxyalkane (PFA)) or metal such as stainless steel.

Next, as illustrated in Fig. 1, the light source unit 20 includes a light source 21 that emits ultraviolet light (for example, light having a peak wavelength of 100 nm to 400 nm), a housing 22 that houses the light source 21, and an ultraviolet light transmission window portion 23 (for example, quartz) attached to the housing 22 so as to be opposed to the second end 12 of the first flow path unit 10.

The kind of the light source 21 is not limited to a particular one, and examples thereof include semiconductor emitting elements such as a light emitting diode (LED) and a laser diode. Although one light source 21 is provided in the present embodiment, two or more light sources 21 may be provided. Furthermore, the light source 21 is installed at a predetermined position in the housing 22 via a substrate so as to be opposed to the first flow path portion 10.

As illustrated in Fig. 2, ultraviolet light emitted from the light source 21 passes through the ultraviolet light transmission window portion 23 and is output in the direction towards the first flow path portion 10. Along with this, from the light source unit 20, for example, in addition to straight light 211 traveling toward the first end 11 of the first flow path portion 10, wide-angle light 212 traveling obliquely toward the sidewall 13 of the first flow path portion 10 and toward the third flow path 30 is emitted. Note that the surface of the light source unit 20, including the ultraviolet light transmission window portion 23, directly in front of the first flow path portion 10 may hereinbelow be referred to as "an ultraviolet light output surface" in some cases.

The relationship between a width W of the ultraviolet light transmission window portion 23 and an inside diameter R1 of the first flow path portion 10 is preferably W > R1 (the width W of the ultraviolet light transmission window portion 23 is larger than the inside diameter R1 of the first flow path portion 10). By setting the relationship between the width W of the ultraviolet light transmission window portion 23 and the inside diameter R1 of the first flow path portion 10 so as to satisfy the above-mentioned inequality, a fluid flowing through the first flow path portion 10 can also receive ultraviolet light emitted through the ultraviolet light transmission window portion 23 when passing through the first communication portion 121 after getting near the ultraviolet light transmission window portion 23. As a result, the fluid can be irradiated with ultraviolet light for a longer period of time, and the ability to sterilize the fluid can further be enhanced. However, the relationship between the width W of the ultraviolet light transmission window portion 23 and the inside diameter R1 of the first flow path portion 10 is not limited to that stated above.

Next, as illustrated in Fig. 1, the second flow path portion 30 includes a first end 31, a second end 32, and a tubular sidewall 33 and extends in the axial direction (longitudinal direction). Also, the first end 31 of the second flow path portion 30 is provided with a second communication portion 311 that communicates with the third flow path portion 40. When the fluid flowing through the third flow path portion 40 gets near the first end 31, the fluid passes through the second communication portion 311 and flows into the second flow path portion 30 (refer to Fig. 2). Here, the second communication portion 311 according to the present embodiment corresponds to a plurality of communication regions (cut-out communication regions) that are formed by cutting out parts of the first end 31 (connection portion to the light source unit 20) in the direction towards the second end 32. The shapes (sizes) of the plurality of communication regions may be equal to, or different from, one another. However, the mode of the second communication portion 311 is not limited to that stated above.

Furthermore, the second flow path portion 20 according to the present embodiment is made of PTFE, but the material is not limited to that stated above. The material for the second flow path portion 30 may be a resin other than PTFE (for example, FEP or PFA) or metal such as stainless steel.

Next, as illustrated in Fig. 1, the third flow path portion 40 is a tubular member arranged outside of the light source unit 20 in the radial direction. Also, the third flow path portion 40 according to the present embodiment is connected to the first flow path portion 10 and the second flow path portion 30 via a C ring, an O ring, or the like. Accordingly, the flow path 41 connecting the first flow path portion 10 to the second flow path portion 30 and covering the outer peripheral region of the light source unit 20 is formed.

That is, after the fluid flowing through the first flow path portion 10 gets near the light source unit 20, the fluid flows along the ultraviolet light output surface of the light source unit 20 and flows into the third flow path portion 40. As a result, the fluid flowing from the first flow path portion 10 into the third flow path portion 40 can be irradiated with the wide-angle light 212 of the ultraviolet light output from the light source unit 20 (refer to Fig. 2).

Also, the fluid flowing from the first flow path portion 10 into the third flow path portion 40 passes through the first communication portion 121 (cut-out communication regions). At this time, the fluid flows into the first communication portion 121 in a concentrated manner. Hence, a turbulent flow is generated near the first communication portion 121, and allows the fluid to stay there. Accordingly, the fluid flowing from the first flow path portion 10 into the third flow path portion 40 can be irradiated with ultraviolet light for a long period of time. Due to the above-described effect, the ability to sterilize the fluid can be enhanced.

Furthermore, the fluid flowing from the third flow path portion 40 into the second flow path portion 30 passes through the second communication portion 311 (cut-out communication regions). When this passing of the fluid occurs, the fluid flows into the second communication portion 311 in a concentrated manner. Hence, a turbulent flow is generated near the second communication portion 311, and allows the fluid to stay there. Accordingly, the fluid in the third flow path portion 40 is retained there. Because of this fluid retention, heat generated from the light source 21 can be dissipated efficiently to the outside of the light source unit 20 via the housing 22 that is in contact with the flow path 41.

In addition, the third flow path portion 40 according to the present embodiment is preferably made of PTFE, which has a high reflectivity with respect to ultraviolet light. By using the third flow path portion 40 made of PTFE, the wide-angle light 212 reaching the third flow path portion 40 can be reflected repeatedly (refer to Fig. 2). Accordingly, the fluid flowing through the third flow path portion 40 can be irradiated with the wide-angle light 212 multiple times, and so the ability to sterilize the fluid can further be enhanced.

However, the material for the third flow path portion 40 is not limited to that stated above. Examples of the other materials include resins other than PTFE (for example, FEP, PFA, and polypropylene (PP)), and metal such as stainless steel.

Incidentally, as illustrated in Fig. 3(d), the cut-out communication regions in the first communication portion 121 and the cut-out communication regions in the second communication portion 311 are arranged alternately in the circumferential direction. More specifically, when the lateral cross-section of the first flow path portion 10, which includes the first communication portion 121 (all of the cut-out communication regions), and the lateral cross-section of the second flow path portion 40, which includes the second communication portion 311 (all of the cut-out communication regions), are combined (combined cross-sectional view in Fig. 3(d)), the first communication portion 121 and the second communication portion 311 are arranged side by side in the circumferential direction (for example, clockwise) in order of the first communication portion 121a, the second communication portion 311a, the first communication portion 121b, the second communication portion 311b, ... the first communication portion 121h, and the second communication portion 311h.

Here, the flow path 41 of the third flow path portion 40 has an annular shape in a lateral cross-sectional view (refer to Fig. 3(b)). For this reason, the fluid that has passed through the first communication portion 121 (for example, the fluid that has passed through the first communication portion 121a) can flow into a freely-selected second communication portion 311 (any of the second communication portions 311a to 311h). When this inflow occurs, each of the second communication portions 311a to 311h is a predetermined distance, in the circumferential direction, away from the first communication portion 121a (the same applies to the other first communication portions 121b to 121h). As a result, the short path of the fluid from the first flow path portion 10 to the second flow path portion 30 via the third flow path portion 40 can be prevented effectively. Therefore, it is possible to allow the fluid to stay near the cut-out communication regions in the first communication portion 121. Accordingly, the fluid can be irradiated with ultraviolet light for a long period of time, and the ability to sterilize the fluid can further be enhanced.

### [Modification Example 1]

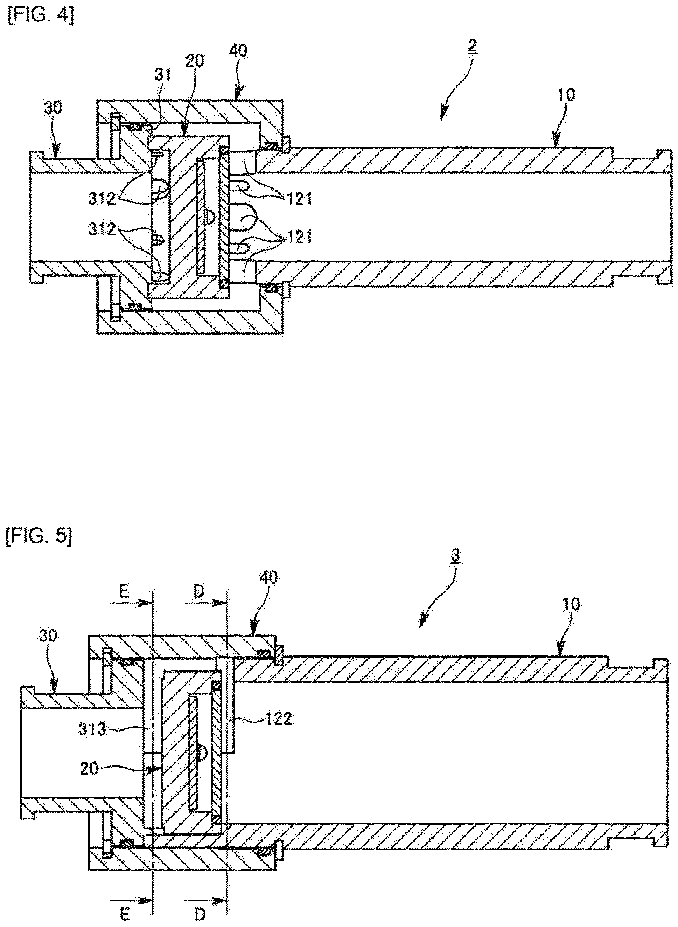

Next, referring to Fig. 4, Modification Example 1 (fluid sterilization device 2) of the fluid sterilization device according to the present embodiment will be described. Here, Fig. 4 is a vertical cross-sectional view of the fluid sterilization device 2. As illustrated in Fig. 4, in the fluid sterilization device 2, a second communication portion 312 (cut-out communication regions) is formed by cutting out a part of the connection portion between the light source unit 20 and the second flow path portion 30 (first end 31) in the direction towards the light source unit 20. That is, the second communication portion 312 is provided on the side facing the light source unit 20. In this respect, Modification Example 1 differs from the basic example (fluid sterilization device 1), in which the second communication portion 311 is provided in the second flow path portion 30.

### [Modification Example 2]

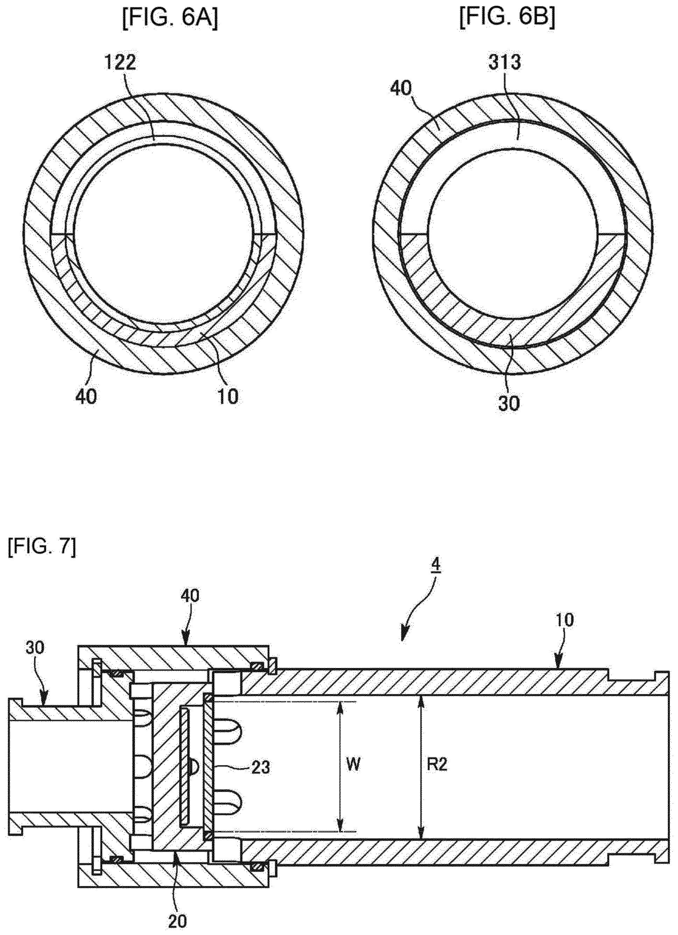

Next, referring to Figs. 5 and 6, Modification Example 2 (fluid sterilization device 3) of the fluid sterilization device according to the present embodiment will be described. Here, Fig. 5 is a vertical cross-sectional view of the fluid sterilization device 3. Also, Fig. 6(a) is a cross-sectional view taken along line D-D in Fig. 5 (a lateral cross-sectional view of the first flow path portion 10), and Fig. 6(b) is a cross-sectional view taken along line E-E in Fig. 5 (a lateral cross-sectional view of the second flow path portion 30).

As illustrated in Figs. 5 and 6, a first communication portion 122 provided at the second end 12 of the first flow path portion 10 corresponds to a semi-circular arc-like communication region (the length (angle) of the arc is not limited to the illustrated one). Also, a second communication portion 313 provided at the first end 31 of the second flow path portion 30 corresponds to a semi-circular arc-like communication region (the length (angle) of the arc is not limited to the illustrated one). In this respect, Modification Example 2 differs from the basic example (fluid sterilization device 1), in which the first communication portion 121 and the second communication portion 311 each corresponds to a plurality of cut-out communication regions.

### [Modification Example 3]

Next, referring to Fig. 7, Modification Example 3 (fluid sterilization device 4) of the fluid sterilization device according to the present embodiment will be described. Here, Fig. 7 is a vertical cross-sectional view of the fluid sterilization device 4. As illustrated in Fig. 7, the relationship between the width W of the ultraviolet light transmission window portion 23 and an inside diameter R2 of the first flow path portion 10 is W ≤ R2 (the width W of the ultraviolet light transmission window portion 23 is equal to or smaller than the inside diameter R2 of the first flow path portion 10). In this respect, Modification Example 3 differs from the basic example (fluid sterilization device 1), in which the relationship between the width W of the ultraviolet light transmission window portion 23 and the inside diameter R1 of the first flow path portion 10 is W > R1.

The embodiment of the present invention has been described above in detail. However, the above-mentioned description has been provided to facilitate understanding of the present invention, not to limit the present invention. The present invention can include one that can be modified or improved without departing from the spirit thereof. Also, the present invention includes equivalents thereof.

### Industrial Applicability

The fluid sterilization device according to the present invention can be used, for example, for an ultraviolet light sterilization device, a water purifier, a water heater, a water pipe, a cooling water circulation device, a water dispenser, or a beverage dispenser. However, the application thereof is not limited to these.

### Reference Signs List

1, 2, 3, 4 ... fluid sterilization device
10 ... first flow path portion
121, 122 ... first communication portion
20 ... light source unit
21 ... light source
22 ... housing
23 ... ultraviolet light transmission window portion
30 ... second flow path portion
311, 312, 313 ... second communication portion
40 ... third flow path portion
41 ... flow path of third flow path portion

## Claims

1. A fluid sterilization device comprising:
a first flow path portion that extends in an axial direction and allows a fluid to pass from a first end to a second end;
a light source unit that is connected to the second end of the first flow path portion and irradiates the fluid with ultraviolet light;
a second flow path portion that is opposed to the first flow path portion with the light source unit interposed between the first flow path portion and the second flow path portion; and
a third flow path portion that is arranged outside of the light source unit in a radial direction and is configured to allow the fluid flowing through the first flow path portion to flow into the second flow path portion, wherein
the first flow path portion or the light source unit includes a first communication portion that communicates with the third flow path portion,
the second flow path portion or the light source unit includes a second communication portion that communicates with the third flow path portion, and
the third flow path portion is any of polymers including polytetrafluoroethylene (PTFE), a perfluoroethylene propene copolymer (FEP), perfluoroalkoxyalkane (PFA), and polypropylene (PP).

2. A fluid sterilization device comprising:
a first flow path portion that extends in an axial direction and allows a fluid to pass from a first end to a second end;
a light source unit that is connected to the second end of the first flow path portion and irradiates the fluid with ultraviolet light;
a second flow path portion that is opposed to the first flow path portion with the light source unit interposed between the first flow path portion and the second flow path portion; and
a third flow path portion that is arranged outside of the light source unit in a radial direction and is configured to allow the fluid flowing through the first flow path portion to flow into the second flow path portion, wherein
the first flow path portion or the light source unit includes a first communication portion that communicates with the third flow path portion,
the second flow path portion or the light source unit includes a second communication portion that communicates with the third flow path portion, and
the first communication portion includes a cut-out communication region that is formed by cutting out a part of a connection portion between the first flow path portion and the light source unit.

3. The fluid sterilization device according to claim 2, wherein:
the first communication portion includes a plurality of the cut-out communication regions;
the second communication portion includes a plurality of cut-out communication regions that are formed by cutting out parts of a connection portion between the second flow path portion and the light source unit; and
the cut-out communication regions in the first communication portion and the cut-out communication regions in the second communication portion are arranged alternately in a circumferential direction.

4. The fluid sterilization device according to any one of claims 1 to 3, wherein:
the light source unit further includes an ultraviolet light transmission window portion opposed to the second end of the first flow path unit; and
a width of the ultraviolet light transmission window portion is larger than an inside diameter of the first flow path portion.
